# EUROPEAN PATENT SPECIFICATION

(11) **EP 1 075 262 B1**
(45) Date of publication and mention of the grant of the patent: **10.08.2005**
(21) Application number: 99921682.3
(22) Date of filing: 05.05.1999
(51) Int. Cl.: A61K 31/44, A61K 31/4439, A61P 1/04, A61P 17/06

(54) **DESMETHYLPANTOPRAZOLE COMPOSITIONS AND METHODS**
DESMETHYLPANTOPRAZOL-ZUSAMMENSETZUNGEN UND VERFAHREN
COMPOSITIONS DE DEMETHYLPANTOPRAZOLE ET PROCEDES LES CONCERNANT

(30) Priority: 05.05.1998 US 84249 P
(43) Date of publication of application: 14.02.2001
(73) Proprietor: Sepracor Inc., Marlborough, Massachusetts 01752 (US)
(72) Inventor: YELLE, William, E., Brewster, MA 02631 (US)
(74) Representative: Lock, Graham James
(86) International application number: PCT/US1999/009815
(87) International publication number: WO 1999/056748

(56) References cited:
- WO-A-94/24867
- WO-A-94/25028
- WO-A-95/18612
- AT-B- 394 368
- MEYER U.A.: "Interaction of proton pump inhibitors with cytochromes P450: Consequences for drug interactions." YALE JOURNAL OF BIOLOGY AND MEDICINE, (1996) 69/3 (203-209). , XP002117370
- HUBER R ET AL: "Pharmacokinetics of pantoprazole in man." INTERNATIONAL JOURNAL OF CLINICAL PHARMACOLOGY AND THERAPEUTICS, (1996 MAY) 34 (5) 185-94. , XP002117371
- R.L. LINS ET AL.: "PHARMACOKINETICS OF THE PROTON PUMP INHIBITOR PANTOPRAZOLE IN PATIENTS WITH SEVERE RENAL IMPAIRMENT" GASTROENTEROLOY, vol. 106, no. 4, 1994, page 126 XP002117372

## Description

### FIELD OF THE INVENTION

This invention relates to compositions of matter containing desmethylpantoprazole. The invention also relates to medicaments for treating and preventing ulcers, treating other conditions related to gastric hypersecretion, and treating psoriasis.

### BACKGROUND OF THE INVENTION

Pantoprazole **I** is an orally active, potent, 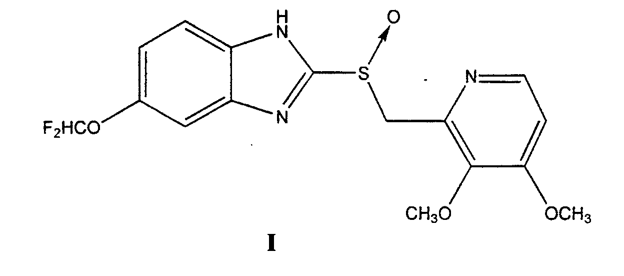 irreversible inhibitor of H⁺,K⁺-ATPase. It is not presently available for human therapy in the United States, but it is available elsewhere throughout the world in the form of delayed release capsules from Byk Gulden Lomberg. The compound is one of the class of compounds known as gastric "proton pump" inhibitors. These compounds are weak organic bases which diffuse passively from the plasma into the acid-containing intracellular canaliculi of gastric parietal cells. At the low pH found in the lumen of these canaliculi, the protonated compounds rearrange to form pyridinium sulfenamides, which react with sulfhydryl groups present on the ATPase localized in the membranes lining the intracellular canaliculi. The alkylation of the sulfhydryl inhibits the ability of the enzyme to catalyze the secretion of H⁺ into the lumen in exchange for K⁺ ions. This inhibition results in an overall reduction in hydrochloric acid secretion by the parietal cells into the cavity of the stomach, thus increasing intragastric pH. As a consequence of reduced acidity in the stomach, the activity of the proteolytic enzyme pepsin is also markedly decreased. Because the proton pump is the final step in acid production and the compounds of this class combine covalently with the associated H⁺,K⁺-ATPase, a profound and prolonged inhibition of gastric acid secretion can be achieved.

Proton pump inhibitors have also been reported as useful in treating psoriasis. [See PCT application WO95/18612]

The Cₘₐₓ of racemic pantoprazole is observed at about 2 to 3 hours in humans after administration of an enteric-coated tablet, and the serum half-life is about 1 to 1.5 hours, although this is variable, depending on the subject's age and liver function, as discussed below. The main serum metabolite is 4'-desmethylpantoprazole, whose Chemical Abstracts index name is 2-[[[5-(difluoromethoxy)-1H-benzimidazol-2-yl]sulfinyl]methyl]-3-methoxy-4-pyridinol, hereinafter referred to as desmethylpantoprazole **II**. 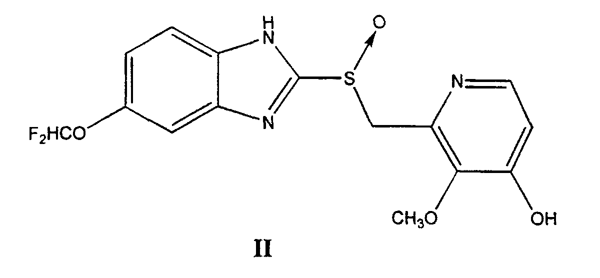 Desmethylpantoprazole is shown above in the "enol" form; the person of skill will recognize that such compounds exist as tautomers and that the corresponding "keto" form is similarly intended to be encompassed within the term "desmethylpantoprazole". Desmethyl-pantoprazole is formed by cytochrome P450 2C 19 (CYP2C 19). CYP2C 19, the S-mephenytoin hydroxylase, is polymorphically expressed in the human population. The mutant allele constitutes the recessive trait. Homozygous carriers of the mutation completely lack CYP2C 19 and are referred to as poor metabolizers (PM's); persons homozygous and heterozygous for the "normal" allele are extensive metabolizers (EM's).

It would be desirable to find a compound with the advantages of pantoprazole which would provide a more predictable dosage regimen in the patient population and that would decrease the chances for drug-drug interactions.

### SUMMARY OF THE INVENTION

This invention relates to the use of desmethylpantoprazole for the manufacture of a medicament for treating ulcers of the stomach, duodenum and esophagus, gastroesophageal reflux diseases, Zollinger-Ellison Syndrome, and other disorders including those that would benefit from an inhibitory action on gastric acid secretion. Desmethylpantoprazole inhibits the H⁺, K⁺-ATPase associated with the gastric proton pump and the resulting secretion of gastric acid by parietal cells providing therapy in diseases associated with gastric hyperacidity. The invention also relates to a medicament for treating psoriasis using desmethylpantoprazole. Desmethylpantoprazole provides a more predictable dosage regimen in the patient population and decreases the chances for drug-drug interactions by avoiding oxidative metabolism for which the cytochrome P450 2C 19 enzyme system is required.

The invention also relates to certain pharmaceutical compositions containing desmethylpantoprazole.

### DETAILED DESCRIPTION OF THE INVENTION

The active compound of these compositions and methods is desmethylpantoprazole. Racemic desmethylpantoprazole may be prepared as described in Austrian Patent.394368.

Desmethylpantoprazole possesses a center of asymmetry at the sulfoxide sulfur, giving rise to two enantiomers. Throughout the instant disclosure, when the term is not otherwise modified, desmethylpantoprazole includes the (+) enantiomer, the (-) enantiomer and any mixture of the two. The enantiomers of desmethylpantoprazole have not been previously disclosed. The individual enantiomers of desmethylpantoprazole can be obtained by asymmetric oxidation of the thioether precursor and bioreduction of the racemate to eliminate one or the other enantiomer in analogous fashion to the procedure described for lansoprazole in PCT applications WO 9602535 and 9617077.

The literature does not appear to disclose any pharmacological activity associated with racemic desmethylpantoprazole.

It has now been discovered that desmethylpantoprazole is a superior agent for treating ulcers of the stomach, duodenum and esophagus, gastroesophageal reflux diseases, Zollinger-Ellison Syndrome, psoriasis and other disorders, including those that would benefit from an inhibitory action on H⁺,K⁺-ATPase in that it provides this effective treatment while exhibiting a lessened liability toward adverse effects than pantoprazole, a lessened liability toward drug-drug interactions than pantoprazole and a more predictable dosing regimen than pantoprazole. Adverse effects of pantoprazole include hepatocellular neoplasia, gastric carcinoids, headache, diarrhea and skin alterations.

The present invention encompasses the treatment of ulcers, which comprises administering to a human in need of such therapy, an amount of racemic desmethylpantoprazole or its (+) or (-) enantiomer, or a pharmaceutically acceptable salt thereof, said amount being sufficient to alleviate the symptoms of ulcers.

The present invention also encompasses an oral antiulcer composition for the treatment of a human in need of antiulcer therapy, which comprises a pharmaceutically acceptable carrier for oral administration and a therapeutically effective amount of desmethylpantoprazole or a pharmaceutically acceptable salt thereof. Preferably the composition is in the form of a tablet or capsule, and the amount of desmethylpantoprazole in the tablet or capsule is preferably 100-500 mg.

The present invention further encompasses a medicament for treating gastroesophageal reflux disease and of treating conditions caused by or contributed to by gastric hypersecretion. Conditions associated with hypersecretion in humans may include, but are not limited to, Zollinger-Ellison syndrome.

The present invention further encompasses a medicament for treating psoriasis.

Utilizing desmethylpantoprazole results in enhanced dosage predictability and an improved therapeutic index. In particular, desmethylpantoprazole exhibits less variation in the patient population between so-called extensive metabolizers and poor metabolizers than does pantoprazole.

The term "treating ulcers" as used herein means treating, alleviating or palliating such conditions, and thus providing relief from the symptoms of nausea, heartburn, post-prandial pain, vomiting, and diarrhea.

The term "treating gastroesophageal reflux diseases in a human" as used herein means treating, alleviating or palliating the conditions that result from the backward flow of the stomach contents into the esophagus.

The term "treating a condition caused, or contributed to, by gastric hypersecretion in a human" as used herein means treating, alleviating or palliating such disorders associated with hypersecretion, thus providing relief from the symptoms of the aforementioned conditions. Zollinger-Ellison Syndrome is among the conditions caused by or contributed to by hypersecretion.

The term "treating psoriasis" as used herein means treating, alleviating or palliating the condition, and thus providing relief from the symptoms of pruritis, epidermal scaling, itching and burning.

The term "optically pure" as used herein means that the compositions contain at least 90% by weight of one enantiomer and 10% by weight or less of the other. In a more preferred embodiment the term "substantially optically pure" means that the composition contains at least 98% by weight of one enantiomer, and 2% or less of the opposite enantiomer. In the most preferred embodiment, the term "substantially optically pure" as used herein means that the composition contains greater than 99% by weight of a single enantiomer. These percentages are based upon the total amount of desmethylpantoprazole in the composition.

The magnitude of a prophylactic or therapeutic dose of desmethylpantoprazole in the acute or chronic management of disease will vary with the severity of the condition to be treated and the route of administration. The dose and perhaps the dose frequency will also vary according to the age, body weight and response of the individual patient. In general, the total daily dose range for desmethylpantoprazole for the conditions described herein is from 50 mg to 1500 mg in single or divided doses. Preferably a daily dose range should be 500 mg to 1000 mg in single or divided doses. In managing the patient, the therapy should be initiated at a lower dose, perhaps at 50 mg and increased up to 1000 mg or higher depending on the patient's global response. It is further recommended that children and patients over 65 years and those with impaired renal or hepatic function, initially receive low doses, and that they be titrated based on individual response(s) and blood level(s). It may be necessary to use dosages outside these ranges in some cases as will be apparent to those skilled in the art. Further, it is noted that the clinician or treating physician will know how and when to interrupt, adjust, or terminate therapy in conjunction with individual patient response. The terms "an amount sufficient to alleviate or palliate ulcers" "an amount sufficient to alleviate the symptoms of gastroesophageal reflux", "an amount sufficient to alleviate gastric hypersecretion" and "an amount sufficient to treat psoriasis" are encompassed by the above-described dosage amounts and dose frequency schedule.

The relative activity, potency and specificity of desmethylpantoprazole both as a gastric antisecretory agent and as a plasma gastrin elevating agent can be determined by a pharmacological study in animals according to the method of Decktor et al. [J. Pharmacol. Exp. Ther. 249, 1-5 (1989)]. The test provides an estimate of relative activity, potency and, through a measure of specificity, an estimate of therapeutic index. Fasted rats, implanted with a gastric cannula, receive single oral or parenteral doses of pantoprazole, (+) desmethylpantoprazole, (-) desmethylpantoprazole or racemic desmethylpantoprazole, 1 hour before collection of gastric juice over a four hour period. Acid output and pH are then determined on each sample. Dose response evaluations are performed with each compound to determine the lowest dose which inhibits acid output by at least 95% and maintains gastric pH above 7.0. Plasma gastrin levels are then determined in a second group of rats treated with the doses selected in the first series of tests. Blood samples are taken for analyses over the five hour period after dosing, and both peak level as well as area-under-the-curve analyses of the gastrin responses are made. These responses are then analyzed statistically using Student's "t" test to assess whether equivalent antisecretory doses show differences in gastrin responses.

The differential metabolism among patient populations can be determined by a clinical study in humans according to the method of Rost et al. [Hepatology 23, 1491-1497 (1996)]. The test provides a measure of metabolism in EM's and PM's and thereby a correlation with dose predictability.

A substitute for the clinical study is provided by the method of Karam et al. [Drug Metab. Dispos. 24, 1081-1087 (1996)]: Cloning of CUP2C cDNAs into the yeast vector pAAH5 and expression of the corresponding proteins in yeast is accomplished as described by Goldstein et. al. [Biochemistry 33, 1743-1752 (1994)]. Microsomes from human livers are prepared as described by Raucy and Lasker [Methods Enzymol. 206,577-587 (1991)]. Recombinant yeast microsomes are prepared as described by Iwasaki et al. [J. Biol. Chem. 266, 3380-3382 (1991)]. Rat P450 reductase and human cytochrome *b*₅ are purified as described by Yasukochi and Masters [J. Biol. Chem. 251, 5337-5344 (1976)] and Raucy and Lasker [Human Drug Metabolism: From Molecular Biology to Man (E.H. Jeffrey, ed.) 23-33, CRC Press, Boca Raton, FL, (1993)]. Microsomal protein content is determined by the method of Lowry et al. [J. Biol. Chem. 193, 265-275 (1951)]. The P450 content of human liver and recombinant yeast microsomes is determined from reduced carbon monoxide difference spectra according to the method of Omura and Sato [J. Biol. Chem. 239, 2370-2378 (1964)].

Recombinant yeast microsomes containing 10-50 pmol of P450 are incubated in 50 mM 4-(2-hydroxyethyl)-1-piperazineethanesulfonic acid buffer (HEPES; pH 7.4) containing 0.1 mM EDTA, 1.5 mM magnesium chloride, dilauroylphosphatidylcholine (15 mg/50 pmol of P450), rat liver P450 reductase (100 units/10 pmol of P450;; Human Biologics, Phoenix, AZ), human cytochrome *b*₅ (20 units/10 pmol of P450), and the specified concentration of test compound, in a final volume of 0.25 mL. The reconstituted mixture is preincubated in a shaking water bath at 37°C for 5 min and then placed on ice. Purified radiolabelled pantoprazole and desmethylpantoprazole are mixed with unlabeled material to obtain the desired specific activity, dissolved in methanol (final methanol concentration in the incubation mixture, ≤1%), and added to the incubation mixture at the specified final concentration. The mixture is preincubated in a shaking water bath at 37°C for 5 min. The reaction is initiated by addition of 2 mM NADPH, and incubations are carried out for 15 min. Reactions are terminated by addition of an equal volume of methanol and stored at -70°C until analysis by HPLC.

Metabolism by 16 human liver microsomal preparations is carried out in 100 mM potassium phosphate buffer (pH 7.4) using 100 µg of microsomal protein and 400 µM OP. Dilauroylphosphatidylcholine, P450 reductase, and cytochrome *b*₅ are omitted from these incubations. The (S)-mephenytoin 4'-hydroxylase activities of the human liver samples are determined, and the Western blot analysis of CYP2C19 proteins in human liver microsomes are as described previously [*Goldstein et al. op. cit*.]. Immunoblots are scanned with a laser densitometer (LKB Instruments).

Separation of test compounds and metabolites by HPLC is performed using an HPLC system on reverse-phase C₅ (5 µm) column. An isocratic solvent system consisting of methanol/acetonitrile/water is used for both purification and metabolite isolation, at a flow rate of 1.1 mL/min. Detection is accomplished using an on-line multiwavelength UV detector at 230 nm and a radiochemical detector.

Concentrations of test compound ranging from 12.5 to 400 µM are used to characterize the kinetics of CYP enzymes and human liver microsomal enzymes, and reactions are carried out for 15 min. The kinetics of the recombinant human P450, as well as those of human liver microsomal sample, are characterized. The apparent *K*_{M} and Vₘₐₓ values for the formation of metabolites by these recombinant human P450s are determined using Michaelis-Menten kinetics.

Any suitable route of administration may be employed for providing the patient with an effective dosage of desmethylpantoprazole. Rectal, parenteral (subcutaneous, intramuscular, intravenous), transdermal, and like forms of administration are possible, but oral administration is preferred. Oral dosage forms include tablets, troches, dispersions, suspensions, solutions, capsules, and the like.

The pharmaceutical compositions of the present invention comprise desmethylpantoprazole or a single enantiomer of desmethylpantoprazole as the active ingredient, or a pharmaceutically acceptable salt thereof, and may also contain a pharmaceutically acceptable carrier, and optionally, other therapeutic ingredients.

The terms "pharmaceutically acceptable salts" or "a pharmaceutically acceptable salt thereof" refer to salts prepared from pharmaceutically acceptable non-toxic bases. Since the compound of the present invention is a weak acid and is unstable at low pH, salts may be prepared from pharmaceutically acceptable non-toxic bases including inorganic and organic bases. Suitable pharmaceutically acceptable base addition salts for the compound of the present invention include metallic salts of aluminum, calcium, lithium, magnesium, potassium, sodium, titanium and zinc or organic salts made from lysine, N,N'-dibenzylethylenediamine, chloroprocaine, choline, diethanolamine, ethylenediamine, meglumine (N-methylglucamine) and procaine. Sodium salts are preferred.

The compositions of the present invention include suspensions, solutions, elixirs or solid dosage forms. Carriers such as starches, sugars, and microcrystalline cellulose, diluents, granulating agents, lubricants, binders, disintegrating agents, and the like are suitable in the case of oral solid preparations (such as powders, capsules, and tablets), and oral solid preparations are preferred over the oral liquid preparations. It has been found that the inclusion of mannitol and of basic salts of calcium and magnesium in the compositions allows the preparation of tablets and capsules that retain good stability. Because of the acid instability of desmethylpantoprazole, it is usually advantageous to coat oral solid dosage forms with an enteric or delayed-release coating. This may be accomplished by standard aqueous or nonaqueous techniques. Oral dosage forms suitable for desmethylpantoprazole are described in US patents 5,035,899; 5,401,512 and 5,045,321 and in PCT applications WO96/01624, WO97/12580 and WO97/25030.

In addition to the common dosage forms set out above, the compounds of the present invention may also be administered by controlled release formulations, which are well known in the art. Compositions suitable for rectal administration are described in European Application 645140.

Pharmaceutical compositions of the present invention suitable for oral administration may be presented as discrete units such as capsules, cachets, or tablets, each containing a predetermined amount of the active ingredient, as a powder or granules, or as a solution or a suspension in an aqueous liquid, a non-aqueous liquid, an oil-in-water emulsion, or a water-in-oil liquid emulsion. Such compositions may be prepared by any of the methods of pharmacy, but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation.

For example, a tablet may be prepared by compression or molding, optionally, with one or more accessory ingredients. Compressed tablets may be prepared by compressing in a suitable machine the active ingredient in a free-flowing form such as powder or granules, optionally mixed with a binder, lubricant, inert diluent, surface active agent or dispersing agent. Molded tablets may be made by molding in a suitable machine, a mixture of the powdered compound moistened with an inert liquid diluent. Desirably, each tablet or capsule contains 100-500 mg of the active ingredient.

An enteric coating, such as the polyacrylate Eudragit L® and Eudragit S® series, is applied, preferably with an aqueous dispersion of the coating polymer. Tablets of other strengths may be prepared by altering the ratio of active ingredient to the excipients or to the final weight of the tablet. Oral, as well as parenteral, sustained release drug delivery systems are well known to those skilled in the art, and general methods of achieving sustained release of orally or parenterally administered drugs are found in any standard pharmacy school textbook, for example Remington: The Science and Practice of Pharmacy. Chapter 94 of the 19th edition of Remington entitled " Sustained-Release Drug Delivery Systems" describes the more common types of oral and parenteral sustained-release dosage forms (pages 1660-1675.).

Formulations for parenteral administration include aqueous and non-aqueous sterile injection solutions which may contain anti-oxidants, buffers, bacteriostats and solutes which render the formulation isotonic with the blood of the intended recipient. Formulations for parenteral administration also include aqueous and non-aqueous sterile suspensions, which may include suspending agents and thickening agents. The formulations may be presented in unit-dose of multi-dose containers, for example sealed ampoules and vials, and may be stored in a freeze-dried (lyophilized) condition requiring only the addition of a sterile liquid carrier, for example saline, phosphate-buffered saline (PBS) or the like, immediately prior to use. Extemporaneous injection solutions and suspensions may be prepared from sterile powders, granules and tablets of the kind previously described.

Formulations for rectal administration may be presented as a suppository with the usual carriers such as cocoa butter or polyethylene glycol.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerin or sucrose and acacia.

Preferred unit dosage formulations are those containing an effective dose, as hereinbelow recited, or an appropriate fraction thereof, of the active ingredient.

The invention is further defined by reference to the following examples describing in detail the preparation of the compositions of the present invention, as well as their utility.

### EXAMPLES

| Example 1 - 250 mg Tablets | |
|---|---|
| Composition per tablet: | |
| desmethylpantoprazole | 250 mg |
| croscarmellose | 60 mg |
| colloidal silicon dioxide | 8 mg |
| magnesium stearate | 1 mg |
| microcrystalline cellulose | 190 mg |
| croscarmellose | 15 mg |
| talc | 10 mg |
| Total | 534 mg |

### Example 1

Desmethylpantoprazole and silicon dioxide are dry mixed, the first portion of croscarmellose is added and the mixture is further dry mixed. The magnesium stearate is added, dry mixed and the mixture is run through a roller compactor and mill. The resulting dry granulate is mixed with the remaining three ingredients and compressed into tablets.

| Example 2 - 200 mg Tablets | |
|---|---|
| Composition per unit dosage: | |
| desmethylpantoprazole | 200 mg |
| pregelatinized starch | 200 mg |
| microcrystalline cellulose | 25 mg |
| povidone | 15 mg |
| coscarmellose | 10 mg |
| magnesium stearate | 3.75 mg |
| FD&C yellow #2 lake | 2.5 mg |
| Water | (5 mL) |
| Total | 456.25 mg |

### Example 2

The ingredients above are mixed well in the proportions shown in a high shear mixer until uniform granules result. The mixture is tray-dried at 40°C under vacuum until the desired consistency is reached. The granules are milled to less than 60 mesh using a screen mill and compressed into tablets.

| Example 3 - Enteric Coating | |
|---|---|
| Enteric coating composition: | |
| Eudragit L-30D | 138 mg (solids 41.4 mg) |
| Talc | 4.1 mg |
| Polyethylene glycol 5000 | 12.4 mg |
| Tween 80 | 2.1 mg |
| Water | 250 µl |

Enteric tablets are produced by coating the tablets obtained in Example 2 with the enteric coating composition shown in a pan coater.

| Example 4 - Aqueous Suspension for Injection | |
|---|---|
| A suspending vehicle is prepared from the following materials: | |
| Polyethylene glycol 4000 | 30 gm. |
| Potassium chloride | 11.2 gm. |
| Polysorbate 80 | 2 gm. |
| Methylparaben | 0.2 gm. |
| Water for injection q.s. | 1000 mL. |

The parabens are added to a major portion of the water and are dissolved therein by stirring and heating to 65° C. The resulting solution is cooled to room temperature and the remainder of the ingredients are added and dissolved. The balance of the water to make up the required volume is then added and the solution sterilized by filtration. The sterile vehicle thus prepared is then mixed with 3 gm of desmethylpantoprazole sodium salt, which has been previously reduced to a particle size less than about 10 microns and sterilized with ethylene oxide gas. The mixture is passed through a sterilized colloid mill and filled under aseptic conditions into sterile containers which are then sealed.

## Claims

1. Use of desmethylpantoprazole or a pharmaceutically acceptable salt thereof in the manufacture of a medicament for treating ulcers.

2. Use of desmethylpantoprazole or a pharmaceutically acceptable salt thereof in the manufacture of a medicament for treating gastroesophageal reflux disease.

3. Use of desmethylpantoprazole or a pharmaceutically acceptable salt thereof in the manufacture of a medicament for treating a condition caused by or contributed to by gastric hypersecretion.

4. Use according to claim 3 wherein said condition is Zollinger-Ellison Syndrome.

5. Use of desmethylpantoprazole or a pharmaceutically acceptable salt thereof in the manufacture of a medicament for treating psoriasis.

6. Use according to any preceding claim wherein desmethylpantoprazole is for oral administration.

7. Use according to any preceding claim wherein the amount of desmethylpantoprazole or a pharmaceutically acceptable salt thereof administered is from 50 mg to 1500 mg per day.

8. Use according to any claims 1 to 5 wherein desmethylpantoprazole is for parenteral administration.

9. Use according to any preceding claim wherein the desmethylpantoprazole is optically pure (+)-desmethylpantoprazole.

10. Use according to any one of claims 1 to 8 wherein the desmethypantoprazole is optically pure (-)-desmethylpantoprazole.

11. Use according to any one of claims 1 to 8 wherein the desmethypantoprazole is racemic desmethylpantoprazole.

12. A medicament comprising a pharmaceutically acceptable carrier for oral or parenteral therapy and desmethylpantoprazole or a pharmaceutically acceptable salt thereof.

13. A medicament according to claim 12 comprising a pharmaceutically acceptable carrier for oral or parenteral therapy and optically pure (+)-desmethylpantoprazole or a pharmaceutically acceptable salt thereof.

14. A medicament according to claim 12 comprising a pharmaceutically acceptable carrier for oral or parenteral therapy and optically pure (-)-desmethylpantoprazole or a pharmaceutically acceptable salt thereof.

15. A medicament according to claim 12 comprising a pharmaceutically acceptable carrier for oral or parenteral therapy and racemic desmethylpantoprazole or a pharmaceutically acceptable salt thereof.

16. A medicament according to any of claims 12 to 15 in the form of a tablet or capsule.

17. Desmethylpantoprazole for use in therapy.

18. Desmethylpantoprazole according to claim 17 for use in treating ulcers, gastroesophageal reflux disease, Zollinger-Ellison Syndrome or other condition caused by or contributed to by gastric hypersecretion.

19. Desmethylpantoprazole according to claim 17 for use in treating psioriasis.

20. Desmethylpantoprazole for use according to any one of claims 17 to 19 by oral administration.

21. Desmethylpantoprazole for use according to claim 20, for administration from 50 mg to 1500 mg per day.

22. Desmethylpantoprazole for use according to any one of claims 17 to 19, wherein the desmethylpantoprazole is (+)- desmethylpantoprazole and is for oral administration.

23. Desmethylpantoprazole for use according to any one of claims 17 to 19, wherein the desmethylpantoprazole is (-)- desmethylpantoprazole and is for oral administration.

24. Desmethylpantoprazole for use according to any one of claims 17 to 19, wherein the desmethylpantoprazole is racemic desmethylpantoprazole and is for oral administration.

## Patentansprüche

1. Verwendung von Desmethylpantoprazol oder einem pharmazeutisch verträglichen Salz davon zur Herstellung eines Medikaments zur Behandlung von Geschwüren.

2. Verwendung von Desmethylpantoprazol oder einem pharmazeutisch verträglichen Salz davon zur Herstellung eines Medikaments zur Behandlung von gastro-ösophagealer Refluxerkrankung.

3. Verwendung von Desmethylpantoprazol oder einem pharmazeutisch verträglichen Salz davon zur Herstellung eines Medikaments zur Behandlung eines Zustands, der durch übermäßig verstärkte Sekretion des Magens verursacht oder an dem übermäßig verstärkte Sekretion des Magens beteiligt ist.

4. Verwendung nach Anspruch 3, wobei der Zustand das Zolliger-Ellison Syndrom ist.

5. Verwendung von Desmethylpantoprazol oder einem pharmazeutisch verträglichen Salz davon zur Herstellung eines Medikaments zur Behandlung von Psoriasis.

6. Verwendung nach einem der vorhergehenden Ansprüche, wobei Desmethylpantoprazol zur oralen Verabreichung vorgesehen ist.

7. Verwendung nach einem der vorhergehenden Ansprüche, wobei die verabreichte Menge von Desmethylpantoprazol oder einem pharmazeutisch verträglichen Salz davon von 50 mg bis 1500 mg pro Tag beträgt.

8. Verwendung nach einem der Ansprüche 1 bis 5, wobei Desmethylpantoprazol zur parenteralen Verabreichung vorgesehen ist.

9. Verwendung nach einem der vorstehenden Ansprüche, wobei das Desmethylpantoprazol optisch reines (+)-Desmethylpantoprazol ist.

10. Verwendung nach einem der Ansprüche 1 bis 8, wobei das Desmethylpantoprazol optisch reines (-)-Desmethylpantoprazol ist.

11. Verwendung nach einem der Ansprüche 1 bis 8, wobei das Desmethylpantoprazol racemisches Desmethylpantoprazol ist.

12. Medikament, umfassend einen pharmazeutisch verträglichen Träger zur oralen oder parenteralen Therapie und Desmethylpantoprazol oder ein pharmazeutisch verträgliches Salz davon.

13. Medikament nach Anspruch 12, umfassend einen pharmazeutisch verträglichen Träger zur oralen oder parenteralen Therapie und optisch reines (+)-Desmethylpantoprazol oder ein pharmazeutisch verträgliches Salz davon.

14. Medikament nach Anspruch 12, umfassend einen pharmazeutisch verträglichen Träger zur oralen oder parenteralen Therapie und optisch reines (-)-Desmethylpantoprazol oder ein pharmazeutisch verträgliches Salz davon.

15. Medikament nach Anspruch 12, umfassend einen pharmazeutisch verträglichen Träger zur oralen oder parenteralen Therapie und ein racemisches Desmethylpantoprazol oder ein pharmazeutisch verträgliches Salz davon.

16. Medikament nach einem der Ansprüche 12 bis 15 in der Form einer Tablette oder Kapsel.

17. Desmethylpantoprazol zur Verwendung in einer Therapie.

18. Desmethylpantoprazol nach Anspruch 17 zur Verwendung in der Behandlung von Geschwüren, gastro-ösophagealer Refluxerkrankung, Zolliger-Ellison Syndrom oder einem anderen Zustand, der durch übermäßig verstärkte Sekretion des Magens verursacht oder an dem übermäßig verstärkte Sekretion des Magens beteiligt ist.

19. Desmethylpantoprazol nach Anspruch 17 zur Verwendung in der Behandlung von Psoriasis.

20. Desmethylpantoprazol zur Verwendung nach einem der Ansprüche 17 bis 19 durch orale Verabreichung.

21. Desmethylpantoprazol zur Verwendung nach Anspruch 20 mit einer Verabreichung von 50 mg bis 1500 mg pro Tag.

22. Desmethylpantoprazol zur Verwendung nach einem der Ansprüche 17 bis 19, wobei das Desmethylpantoprazol (+)-Desmethylpantoprazol ist und zur oralen Verabreichung vorgesehen ist.

23. Desmethylpantoprazol zur Verwendung nach einem der Ansprüche 17 bis 19, wobei das Desmethylpantoprazol (-)-Desmethylpantoprazol ist und zur oralen Verabreichung vorgesehen ist.

24. Desmethylpantoprazol zur Verwendung nach einem der Ansprüche 17 bis 19, wobei das Desmethylpantoprazol racemisches Desmethylpantoprazol ist und zur oralen Verabreichung vorgesehen ist.

## Revendications

1. Utilisation de déméthylpantoprazole ou un sel pharmaceutiquement acceptable de celui-ci pour la fabrication d'un médicament pour le traitement des ulcères.

2. Utilisation de déméthylpantoprazole ou un sel pharmaceutiquement acceptable de celui-ci pour la fabrication d'un médicament pour le traitement du reflux gastro-oesophagien pathologique.

3. Utilisation de déméthylpantoprazole ou un sel pharmaceutiquement acceptable de celui-ci pour la fabrication d'un médicament pour le traitement d'un état provoqué par ou auquel contribue l'hypersecrétion gastrique.

4. Utilisation selon la revendication 3 où cet état est le syndrome de Zollinger-Ellison.

5. Utilisation de déméthylpantoprazole ou un sel pharmaceutiquement acceptable de celui-ci pour la fabrication d'un médicament pour le traitement du psoriasis.

6. Utilisation selon l'une quelconque des revendications précédentes où le déméthylpantoprazole est prévu pour une administration par voie orale.

7. Utilisation selon l'une quelconque des revendications précédentes où la quantité de déméthylpantoprazole ou un sel pharmaceutiquement acceptable de celui-ci est administrée en quantités de 50 mg à 1500 mg par jour.

8. Utilisation selon l'une quelconque des revendications 1 à 5 où le déméthylpantoprazole est prévu pour une administration par voie parentérale.

9. Utilisation selon l'une quelconque des revendications précédentes où le déméthylpantoprazole est un déméthylpantoprazole (+) est optiquement pur.

10. Utilisation selon l'une quelconque des revendications 1 à 8 où le déméthylpantoprazole est un déméthylpantoprazole (-) optiquement pur.

11. Utilisation selon l'une quelconque des revendications 1 à 8 où le déméthylpantoprazole est un déméthylpantoprazole racémique.

12. Un médicament comprenant un véhicule pharmaceutiquement acceptable pour une thérapie par voie orale ou parentérale et du déméthylpantoprazole ou un sel pharmaceutiquement acceptable de celui-ci.

13. Un médicament selon la revendication 12 comprenant un véhicule pharmaceutiquement acceptable pour une thérapie par voie orale ou parentérale et du déméthylpantoprazole (+) optiquement pur ou un sel pharmaceutiquement acceptable de celui-ci.

14. Un médicament selon la revendication 12 comprenant un véhicule pharmaceutiquement acceptable pour une thérapie par voie orale ou parentérale et du déméthylpantoprazole (-) optiquement pur ou un sel pharmaceutiquement acceptable de celui-ci.

15. Un médicament selon la revendication 12 comprenant un véhicule pharmaceutiquement acceptable pour une thérapie par voie orale ou parentérale et du déméthylpantoprazole racémique ou un sel pharmaceutiquement acceptable de celui-ci.

16. Un médicament selon l'une quelconque des revendications 12 à 15 sous forme de comprimés ou de capsules.

17. Déméthylpantoprazole à utiliser en thérapeutique.

18. Déméthylpantoprazole selon la revendication 17 à utiliser pour le traitement d'ulcères, du reflux gastro-oesophagien pathologique, du syndrome de Zollinger-Ellison ou d'autres états provoqués par ou auxquels contribue l'hypersecrétion gastrique.

19. Déméthylpantoprazole selon la revendication 17 à utiliser pour le traitement du psoriasis.

20. Déméthylpantoprazole à utiliser selon l'une quelconque des revendications 17 à 19 par voie orale.

21. Déméthylpantoprazole à utiliser selon la revendication 20 pour une administration de 50 mg à 1500 mg par jour.

22. Déméthylpantoprazole à utiliser selon l'une quelconque des revendications 17 à 19 où le déméthylpantoprazole est du déméthylpantoprazole (+) et est prévu pour une administration par voie orale.

23. Déméthylpantoprazole à utiliser selon l'une quelconque des revendications 17 à 19 où le déméthylpantoprazole est du déméthylpantoprazole (-) et est prévu pour une administration par voie orale.

24. Déméthylpantoprazole à utiliser selon l'une quelconque des revendications 17 à 19 où le déméthylpantoprazole est du déméthylpantoprazole racémique et est prévu pour une administration par voie orale.
